# EUROPÄISCHE PATENTSCHRIFT

(11) **EP 0 775 691 B1**
(45) Veröffentlichungstag und Bekanntmachung des Hinweises auf die Patenterteilung: **26.01.2000**
(21) Anmeldenummer: 96118232.6
(22) Anmeldetag: 14.11.1996
(51) Int. Cl.: C07C 253/20

(54) **Verfahren zur Herstellung von Nitrilen**
Process for the preparation of nitriles
Procédé de préparation de nitriles

(30) Priorität: 21.11.1995 CH 329095
(43) Veröffentlichungstag der Anmeldung: 28.05.1997
(73) Patentinhaber: F. HOFFMANN-LA ROCHE AG, 4070 Basel (CH)
(72) Erfinder: Bonrath, Werner, 79115 Freiburg (DE); Pauling, Horst, 4103 Bottmingen (CH)
(74) Vertreter: Kellenberger, Marcus, Dr.

(56) Entgegenhaltungen:
- US-A- 4 908 452
- SOVIET INVENTIONS ILLUSTRATED Section Ch, Week 8243 8.Dezember 1982 Derwent Publications Ltd., London, GB; Class A41, AN 92129e XP002025751 & SU 891 650 A (GORKI POLY) , 25.Dezember 1981
- CHEMICAL ABSTRACTS, vol. 86, no. 1, 1977 Columbus, Ohio, US; abstract no. 5116c, Spalte 1; XP002025750 & ZH. ORG. KHIM., Bd. 12, Nr. 7, 1976, Seite 1588 E.A. LYANDAEV ET AL:

## Beschreibung

Die vorliegende Erfindung betrifft ein neues Verfahren zur Herstellung von Nitrilen durch Dehydratisierung der entsprechenden Amide.

Es sind in den letzten Jahren mehrere Verfahren zur Herstellung von Nitrilen durch Dehydratisierung der entsprechenden Amide bekannt geworden. Bei einem dieser bekannten Verfahren handelt es sich beispielsweise um die Dehydratisierung in Gegenwart von Phosphorpentoxid [T. Rinderspacher und B. Prijs, Helv. Chim. Acta, **43**, 1522-1530 (1960)]. Der Nachteil dieses Verfahrens ist jedoch die geringe Produktausbeute, was auf die bei dieser Reaktion sehr leicht auftretende Verkohlung zurückzuführen ist.

Der vorliegenden Erfindung liegt nun die Aufgabe zugrunde, ein Verfahren zur Herstellung von Nitrilen aus Amiden durch Dehydratisierung zur Verfügung zu stellen, gemäss welchem das Nitril in kurzer Reaktionszeit, unter milden Reaktionsbedingungen und in hoher Ausbeute erhalten wird.

Das erfindungsgemässe Verfahren ist dadurch gekennzeichnet, dass die Dehydratisierung in Gegenwart eines Adduktes aus Schwefeltrioxid und einem Amin in einem basischen Reaktionsmedium durchgeführt wird. Das Amin dieses Adduktes ist insbesondere ein tertiäres Amin.

Zur Bildung des Schwefeltrioxid-Amin-Adduktes sind als Amine sowohl aliphatische Amine als auch Stickstoff enthaltende heteroaromatische Verbindungen geeignet.

Unter den aliphatischen Aminen können im Rahmen der vorliegenden Erfindung insbesondere Trialkylamine mit geradkettigen oder verzweigten Alkylresten enthaltend 1 bis 20 Kohlenstoffatome verwendet werden. Beispiele solcher Alkylreste sind Methyl, Ethyl, n-Propyl, Isopropyl, n-Butyl, Isobutyl, tert.Butyl, n-Pentyl, n-Hexyl und n-Octyl. Bei solchen Trialkylaminen können die Alkylreste gleich oder verschieden sein. Beispiele dieser Trialkylamine - und zugleich bevorzugte Trialkylamine - sind Triethylamin bzw. Diisopropylethylamin.

Bei den Stickstoff enthaltenden heteroaromatischen Verbindungen handelt es sich insbesondere um Heterozyklen, deren Ring mindestens ein Stickstoffatom aufweist. Beispiele sind Pyridin und Pyridinderivate, wie Picolin und Chinolin. Solche Stickstoff enthaltende heteroaromatische Verbindungen können an einen polymeren Träger gebunden sein. Die bevorzugte derartige Verbindung ist Pyridin. Als Beispiel des an einen polymeren Träger gebundenen Pyridins sei Poly-(4-vinyl-pyridin) genannt, dessen Addukt mit Schwefeltrioxid käuflich ist (Fluka Chemie AG, CH-9471 Buchs, Schweiz).

Vorteilhaft ist bei der Verwendung des Schwefeltrioxid-Amin-Adduktes als Dehydratisierungsreagens im erfindungsgemässen Verfahren dessen einfache Herstellung aus den Edukten Schwefeltrioxid und Amin sowie die einfachere Handhabung gegenüber dem aggressiven Schwefeltrioxid, wenn dieses allein verwendet wird. Die Addukte sind zum Teil bekannt und in ein paar Fällen käuflich. Sie können leicht durch Einleitung des Schwefeltrioxids in das verdünnte Amin hergestellt werden, wobei als Verdünnungsmittel beispielsweise Methylenchlorid verwendet werden kann.

Zur Dehydratisierung wird das Amid mit dem Dehydratisierungsreagens zweckmässigerweise bei Raumtemperatur oder höheren Temperaturen bis etwa 120 °C, vorzugsweise bei Temperaturen von etwa 50°C bis etwa 100 °C, besonders bevorzugt bei Temperaturen von etwa 70 °C bis etwa 90 °C, umgesetzt.

Die Dehydratisierung wird in einem basischen Reaktionsmedium durchgeführt. Als Reaktionsmedium können dieselben Amine eingesetzt werden, die zur Bildung des Dehydratisierungsreagenses verwendet werden. Als besonders vorteilhaft hat sich die Dehydratisierung mit dem Schwefeltrioxid-Triethylamin-Addukt in Triethylamin erwiesen. Gewünschtenfalls kann ein Gemisch des Amins mit einem organischen Lösungsmittel als Reaktionsmedium verwendet werden, wobei sich als organische Lösungsmittel insbesondere aliphatische oder cyclische Ether, z.B. tert.Butylmethylether bzw. Tetrahydrofuran oder Dioxan; aliphatische Nitrile, z.B. Acetonitril; sowie aromatische Kohlenwasserstoffe, z.B. Toluol, eignen. Vorzugsweise wird allerdings kein organisches Lösungsmittel verwendet.

Wird ein festes Amid umgesetzt, so wird das Amid zweckmässigerweise im basischen Reaktionsmedium suspendiert.

Das Molverhältnis von Amid zu Dehydratisierungsreagens beträgt zweckmässigerweise etwa 1:1 bis etwa 1:10, vorzugsweise etwa 1:1 bis etwa 1:4, besonders bevorzugt etwa 1:1 bis etwa 2,5.

Nach beendeter Reaktion kann die Aufarbeitung zur Isolierung des Rohprodukts nach geeigneten Methoden der organischen Chemie erfolgen. Zur Abtrennung des Amins kann beispielsweise das Amin mit Chlorwasserstoff in ein Hydrochlorid überführt werden, wobei der Chlorwasserstoff als verdünnte Salzsäure (z.B. 25%ige Salzsäure), als konzentrierte Salzsäure oder sogar als gasförmiger Chlorwasserstoff eingesetzt werden kann. Die Isolierung des Rohprodukts kann auch destillativ erfolgen.

Das erfindungsgemässe Verfahren ermöglicht die Herstellung von Nitrilen aus Amiden unter milden Reaktionsbedingungen und in hohen Ausbeuten. Es eignet sich sowohl für die Dehydratisierung aliphatischer Amide als auch aromatischer und heteroaromatischer Amide.

Als aliphatische Amide können z.B. geradkettige oder verzweigte Alkylamide, wie Hexanamid, eingesetzt werden.

Als aromatische Amide können z.B. 2-Naphthalincarbonsäureamid, Benzamid und substituierte Benzamide, wie 4-Chlorbenzamid oder 3,5-Difluorbenzamid, eingesetzt werden.

Als heteroaromatisches Amid kann z.B. Nicotinsäureamid eingesetzt werden.

Besonders geeignet ist das Verfahren zur Herstellung von 5-Cyano-4-niederalkyl-oxazolen, wie 5-Cyano-4-methyl-oxazol, ein wertvolles Zwischenprodukt bei der Synthese von Pyridoxin (Vitamin B₆).

Die folgenden Beispiele erläutern das erfindungsgemässe Verfahren näher, sollen jedoch keinerlei Einschränkung darstellen.

### Beispiel 1

### Herstellung von 5-Cyano-4-methyl-oxazol

### a) Dehydratisierungsreagens: Triethylaminschwefeltrioxid

Eine Suspension von 200 mmol 5-Carbamoyl-4-methyl-oxazol und 500 mmol Triethylaminschwefeltrioxid in 200 ml Triethylamin wird 6 Stunden bei 89 °C gerührt.

Zur Aufarbeitung wird das in einem Eisbad auf 0 °C gekühlte Reaktionsgemisch mit 150 ml Methyltertiärbutylether versetzt. Zur Neutralisation wird konzentrierte Salzsäure bis zum Erreichen des pH-Wertes 7 zugetropft. Der resultierende Niederschlag wird abfiltriert und mit 100 ml Methyltertiär-butylether gewaschen. Die Phasen des Filtrats werden getrennt, und die Wasserphase zweimal mit je 50 ml Methyltertiärbutylether extrahiert. Die vereinigten organischen Phasen werden einmal mit gesättigter Natriumhydrogencarbonatlösung und einmal mit gesättigter Natriumchloridlösung gewaschen, über wasserfreiem Natriumsulfat getrocknet und filtriert. Schliesslich wird das Filtrat am Rotationsverdampfer bei 40 °C und 500 mbar eingeengt.
Ausbeute: 93 % der Theorie, gaschromatographisch bestimmt.

### b) Dehydratisierungsreagens: Diisopropylethylaminschwefeltrioxid

Eine Suspension von 200 mmol 5-Carbamoyl-4-methyl-oxazol und 500 mmol Diisopropylethylaminschwefeltrioxid in 200 ml Triethylamin wird 6 Stunden bei 89 °C gerührt. Die Aufarbeitung erfolgt wie unter a) beschrieben.
Ausbeute: 92 % der Theorie.

### c) Dehydratisierungsreagens: Pyridinschwefeltrioxid

Eine Suspension von 200 mmol 5-Carbamoyl-4-methyl-oxazol und 500 mmol Pyridinschwefeltrioxid in 200 ml Triethylamin wird 6 Stunden bei 89°C gerührt. Die Aufarbeitung erfolgt wie unter a) beschrieben.
Ausbeute: 45% der Theorie.

### Beispiel 2

### Herstellung von Hexanonitril

Eine Suspension von 50 mmol n-Hexanamid und 125 mmol Triethylaminschwefeltrioxid in 75 ml Triethylamin wird 6 Stunden bei 89 °C gerührt. Die Aufarbeitung erfolgt wie unter Beispiel 1a) beschrieben
Ausbeute: 86% der Theorie.

### Beispiel 3

### Herstellung von 4-Chlorbenzonitril

Eine Suspension von 50 mmol 4-Chlorbenzamid und 125 mmol Triethylaminschwefeltrioxid in 75 ml Triethylamin wird 6 Stunden bei 89 °C gerührt. Die Aufarbeitung erfolgt wie unter Beispiel 1a) beschrieben.
Ausbeute: 92% der Theorie.

### Beispiel 4

### Herstellung von 3,5-Difluorbenzonitril

Eine Suspension von 10 mmol 3,5-Difluorbenzamid und 25 mmol Triethylaminschwefeltrioxid in 30 ml Triethylamin wird 1 Stunde bei 89 °C gerührt. Die Aufarbeitung erfolgt wie unter Beispiel 1a) beschrieben.
Ausbeute: 85.7% der Theorie.

### Beispiel 5

### Herstellung von Naphthalin-2-carbonitril

Eine Suspension von 10 mmol Naphthalincarbonsäureamid und 25 mmol Triethylaminschwefeltrioxid in 30 ml Triethylamin wird 2 Stunden bei 89 °C gerührt. Die Aufarbeitung erfolgt wie unter Beispiel 1a) beschrieben.
Ausbeute: 91% der Theorie.

### Beispiel 6

### Herstellung von 3-Cyanopyridin.

Eine Suspension von 10 mmol Nicotinsäureamid und 25 mmol Triethylaminschwefeltrioxid in 30 ml Triethylamin wird 2 Stunden bei 89 °C gerührt. Die Aufarbeitung erfolgt wie unter Beispiel 1a) beschrieben.
Ausbeute: 95% der Theorie.

### Beispiel 7

### Herstellung von 5-Ethoxy-4-methyloxazol-2-nitril

Eine Suspension von 10 mmol 5-Ethoxy-4-methyloxazol-2-carboxamid und 25 mmol Triethylaminschwefeltrioxid in 30 ml Triethylamin wird 6 Stunden bei 89 °C gerührt. Die Aufarbeitung erfolgt wie unter Beispiel 1a) be-schrieben. Ausbeute: 76% der Theorie.

## Patentansprüche

1. Verfahren zur Dehydratisierung von Amiden zu Nitrilen, dadurch gekennzeichnet, dass die Dehydratisierung in Gegenwart eines Adduktes aus Schwefeltrioxid und einem Amin als Dehydratisierungsreagens in einem basischen Reaktionsmedium durchgeführt wird.

2. Verfahren gemäss Anspruch 1, dadurch gekennzeichnet, dass das Amin des Dehydratisierungsreagenses ein tertiäres Amin ist.

3. Verfahren gemäss Anspruch 2, dadurch gekennzeichnet, dass das Amin ein aliphatisches Amin oder eine Stickstoff enthaltende heteroaromatische Verbindung ist.

4. Verfahren gemäss Anspruch 3, dadurch gekennzeichnet, dass das aliphatische Amin ein Trialkylamin ist, dessen Alkylreste je 1 bis 20 Kohlenstoffatome enthalten.

5. Verfahren gemäss Anspruch 3, dadurch gekennzeichnet, dass die heteroaromatische Verbindung Pyridin oder ein Pyridinderivat ist.

6. Verfahren gemäss einem der Ansprüche 1 bis 5, dadurch gekennzeichnet, dass die Dehydratisierung bei Temperaturen von Raumtemperatur bis etwa 120 °C, vorzugsweise von etwa 50 °C bis etwa 100°C, besonders bevorzugt von etwa 70 °C bis etwa 90 °C, durchgeführt wird.

7. Verfahren gemäss einem der Ansprüche 1 bis 6, dadurch gekennzeichnet, dass das Molverhältnis von Amid zu Dehydratisierungsreagens 1:1 bis 1:10, vorzugsweise 1:1 bis 1:4, besonders bevorzugt 1:1 bis 1: 2,5, beträgt.

8. Verfahren gemäss einem der Ansprüche 1 bis 7, dadurch gekennzeichnet, dass ein aliphatisches Amid dehydratisiert wird.

9. Verfahren gemäss einem der Ansprüche 1 bis 7, dadurch gekennzeichnet, dass ein aromatisches Amid dehydratisiert wird.

10. Verfahren gemäss einem der Ansprüche 1 bis 7, dadurch gekennzeichnet, dass ein heteroaromatisches Amid dehydratisiert wird.

11. Verfahren gemäss Anspruch 10, dadurch gekennzeichnet, dass 5-Carbamoyl-4-methyl-oxazol zu 5-Cyano-4-methyl-oxazol dehydratisiert wird.

## Claims

1. A process for the dehydration of amides to nitriles, which process comprises carrying out the dehydration in the presence of an adduct of sulphur trioxide and an amine as the dehydrating reagent in a basic reaction medium.

2. A process according to claim 1, wherein the amine of the dehydrating reagent is a tertiary amine.

3. A process according to claim 2, wherein the amine is an aliphatic amine or a nitrogen-containing heteroaromatic compound.

4. A process according to claim 3, wherein the aliphatic amine is a trialkylamine in which each alkyl residue contains 1 to 20 carbon atoms.

5. A process according to claim 3, wherein the heteroaromatic compound is pyridine or a pyridine derivative.

6. A process according to any one of claims 1 to 5, wherein the dehydration is carried out at temperatures of room temperature to about 120°C, preferably of about 50°C to about 100°C, especially of about 70°C to about 90°C.

7. A process according to any one of claims 1 to 6, wherein the molar ratio of amide to dehydrating reagent is 1:1 to 1:10, preferably 1:1 to 1:4, especially 1:1 to 1:2.5.

8. A process according to any one of claims 1 to 7, wherein an aliphatic amide is dehydrated.

9. A process according to any one of claims 1 to 7, wherein an aromatic amide is dehydrated.

10. A process according to any one of claims 1 to 7, wherein a heteroaromatic amide is dehydrated.

11. A process according to claim 10, wherein 5-carbamoyl-4-methyl-oxazole is dehydrated to 5-cyano-4-methyloxazole.

## Revendications

1. Procédé de déshydratation d'amides en nitriles, caractérisé en ce qu'on conduit la déshydratation en présence d'un produit d'addition d'anhydride sulfurique et d'une amine comme réactif de déshydratation dans un milieu réactionnel basique.

2. Procédé selon la revendication 1, caractérisé en ce que l'amine du réactif de déshydratation est une amine tertiaire.

3. Procédé selon la revendication 2, caractérisé en ce que l'amine est une amine aliphatique ou un composé hétéro-aromatique contenant de l'azote.

4. Procédé selon la revendication 3, caractérisé en ce que l'amine aliphatique est une trialkylamine dont les radicaux alkyle contiennent chacun de 1 à 20 atomes de carbone.

5. Procédé selon la revendication 3, caractérisé en ce que le composé hétéro-aromatique est la pyridine ou un dérivé de pyridine.

6. Procédé selon l'une des revendications 1 à 5, caractérisé en ce qu'on conduit la déshydratation à des températures allant de la température ambiante à environ 120°C, de préférence d'environ 50°C à environ 100°C, de façon particulièrement préférée d'environ 70°C à environ 90°C.

7. Procédé selon l'une des revendications 1 à 6, caractérisé en ce que le rapport molaire de l'amide au réactif de déshydratation est de 1:1 à 1:10, de préférence de 1:1 à 1:4, de façon particulièrement préférée de 1:1 à 1:2,5.

8. Procédé selon l'une des revendications 1 à 7, caractérisé en ce qu'on déshydrate un amide aliphatique.

9. Procédé selon l'une des revendications 1 à 7, caractérisé en ce qu'on déshydrate un amide aromatique.

10. Procédé selon l'une des revendications 1 à 7, caractérisé en ce qu'on déshydrate un amide hétéro-aromatique.

11. Procédé selon la revendication 10, caractérisé en ce qu'on déshydrate le 5-carbamoyl-4-méthyl-oxazole en 5-cyano-4-méthyl-oxazole.
